Europäisches Patentamt

European Patent Office

Office européen des brevets

(11) Publication number: **0 315 459**
**A2**

# (12) EUROPEAN PATENT APPLICATION

(21) Application number: **88310377.2**

(22) Date of filing: **04.11.88**

(51) Int. Cl.⁴: **C 12 P 21/00**
**A 61 K 39/21, C 07 K 15/04**
**//(C12P21/00,C12R1:91)**

(30) Priority: **05.11.87 JP 278290/87**

(43) Date of publication of application:
**10.05.89 Bulletin 89/19**

(84) Designated Contracting States:
**AT BE CH DE ES FR GB GR IT LI LU NL SE**

(71) Applicant: **Kato, Shira**
**23-7, Fujishirodai-4-chome**
**Suita-shi (JP)**

**Ikuta, Kazuyoshi**
**18-1-117, Yamadahigashi-3-chome**
**Suita-shi (JP)**

(72) Inventor: **Kato, Shira**
**23-7, Fujishirodai-4-chome**
**Suita-shi (JP)**

**Ikuta, Kazuyoshi**
**18-1-117, Yamadahigashi-3-chome**
**Suita-shi (JP)**

(74) Representative: **Harrison, David Christopher et al**
**MEWBURN ELLIS & CO 2/3 Cursitor Street**
**London EC4A 1BQ (GB)**

(54) **HIV incomplete particles and a method for production thereof.**

(57) HIV incomplete particles which comprise as main consti-
tuent protein gp 120 or a gp 160-like substance and/or
fragments thereof, and do not comprise or do not substantially
comprise protein encoded by gag gene and/or protein encoded
by pol gene are produced by culturing HIV incomplete
particle-producing cells survived from HIV-infected cells and
recovering the HIV incomplete particles from the culture fluid.
The HIV incomplete particles have no infectivity and are
expected to be effective for preventing AIDS.

## Description

### HIV INCOMPLETE PARTICLES AND A METHOD FOR PRODUCTION THEREOF

The present invention relates to HIV incomplete particles which are free from infectivity and can thus be used as medical drugs such as vaccine, etc. and to a method for production thereof.

The term "HIV" is an abbrebiation of human immunodeficiency virus and refers to a virus which causes AIDS (Acquired Immune Deficiency Syndrome), including HIV-1 (human immunodeficiency virus type 1) such as HTLV-IIIB and LAV-I; and HIV-2. HIV belongs to Retrovirus and is a virus that in general infects mainly CD4 positive cells out of human T-lymphocytes, destroys the cells, induces severe immunodeficient symptoms on patient and finally causes him to die. Development of drugs for treating AIDS or vaccine for preventing infections has been extensively made in the world, and in the background technology of culturing viruses and cells has been considerably progressed. First, assay for HIV-1 have become possible using cultured cells. That is, it has become possible to detect HIV-1, by establishing human T-lymphocyte-derived cell line having a high sensitivity to HIV-1 and measuring inhibition on the cell line growth or denaturation of the cell line caused by infection with HIV-1. Next, in contrast to this cell line, the persistently HIV-1 infected cell line which is capable of growing persistently even though it is infected with HIV-1, has been developed. As is described in, for example, Koyanagi, Y. et al., "Selective cytotoxicity of AIDS virus infection towards HTLV-1-transformed cell lines", Int. J. Cancer, 36, 445-451 (1985), among acute lymphoid leukemia (ALL)-derived cell lines, after infection with HIV-1, many cells become free from death and turn into cells capable of being subcultured while releasing HIV-1. This cell line is important for harvesting infectious HIV-1 stably in a large scale and using the same for measurement of an antibody in the serum of the patient infected with HIV-1 or as a raw material for vaccine, etc. The term "infectivity" or "infectious" as used herein means properties attributed to HIV per se and refers to an ability to bind to cells having high sensitivity to HIV and to be transduced into the cells to replicate thereby to inhibit growth of the cells or finally cause them to die or refers to an ability to denaturate the cells.

Examples of the cells having high sensitivity to HIV include MT-2 cells, MT-4 cells and the like. HIV as a raw material for vaccine is attenuated or inactivated by an appropriate treatment and can be used as live vaccine or inactivated vaccine. On the other hand, an attempt has been made to isolate from HIV constituent components a protein capable of readily inducing the production of an antibody specific thereto in the human serum or to prepare a protein identical with one of HIV constituent components by recombinant DNA technology or chemical synthesis and to use such a protein as so called component vaccine. In this case, among the HIV constituent components, envelope protein is generally selected because of having high immunogenicity and the advantage of effective induction of the antibody.

With regard to incomplete HIV-1, HIV-1 particles of doughnut-type are known. The shape is merely observed as an electron microscopic finding but the doughnut particles are found to be intermingled with complete HIV-1 particles as a part of these particles. It is further reported that any core structure is not observed in the doughnut particles [Nakai MASUYO, "Morphological structure of AIDS virus particles and progress of producing them," Cell Engineering, 5, No. 13, 1137-1145 (1986)].

In case that one wishes to harvest HIV in a large scale and provide HIV for use as drugs, etc., infectivity of HIV would be a serious problem, as a matter of course. Such a problem may well also arise in the case of using HIV as a drug for external diagnosis or a reagent for purposes of research, and a serious problem occurs particularly in the case of administering HIV as a therapeutic agent such as vaccine. In the case of live vaccine comprising, for example, attenuated virus, its pathogenicity might be regained by back or reverse mutation. In the case of inactivated vaccine, there are problems that HIV remains not inactivated and that effects of the vaccine decrease due to inactivation. Further in the component vaccine, the protein used as vaccine is somewhat different from one in naturally occurring HIV, so that the resulting reduction in immunogenicity and the like are obstacles upon practical use. It has thus desired to develop HIV incomplete particles which are as close to natural HIV as possible, have high immunogenicity and have no infectivity and to develop a method for producing these particles in a large scale.

Turning to doughnut particles known to be one of HIV-1 incomplete particles, these particles are understood merely as a certain phenomenon in which core structure is not observed; however, detailed properties as a material are unkown at all. It is yet unclear whether the HIV-1 incomplete particles are infectious or not. The particles appear generally in a trace amount as if they spontaneously generates. Therefore, it has been heretofore difficult to isolate and purify them from the co-existing complete HIV particles and it has been heretofore impossible to prepare the HIV incomplete particles in a large scale.

On the other hand, it is reported that a cellular clone was isolated from an IUdR (5-iodo-2'-deoxyuridine)-induced mass culture that has survived AIDS retro virus infection and the clone 8E5 generated uninfectious defective virus particles that failed to synthesized detectable reverse transcriptase [Thomas M. Falks et al., J. Exp. Med., vol. 164, 280-290 (1986)]. This cell clone synthesizes all major normal structural env and gag proteins except for riverse transcriptase protein.

### SUMMARY OF THE INVENTION

As a result of intensive investigations to eliminate the foregoing problems, the present inventors have found HIV incomplete particles which are free from infectivity and can be stably prepared in a large scale.

Therefore, an object of the present invention is to provide HIV incomplete particles which are not infectious and are utilizable as drugs such as vaccine.

Another object of the present invention is to provide a method for preparing such HIV incomplete particles in a large scale.

In one aspect, the present invention is directed to HIV incomplete particles which comprise protein encoded by env gene and/or fragments thereof as main constituent protein, do not comprise or do not substantially comprise protein encoded by gag gene and/or protein encoded by pol gene and are produced by cells.

In another aspect, the present invention is directed to a method for preparing HIV incomplete particles which comprises infecting cells with HIV, harvesting cells survived thereafter and capable of producing HIV incomplete particles, culturing the cells and recovering the HIV incomplete particles from the culture fluid.

BRIEF DESCRIPTION OF THE DRAWING

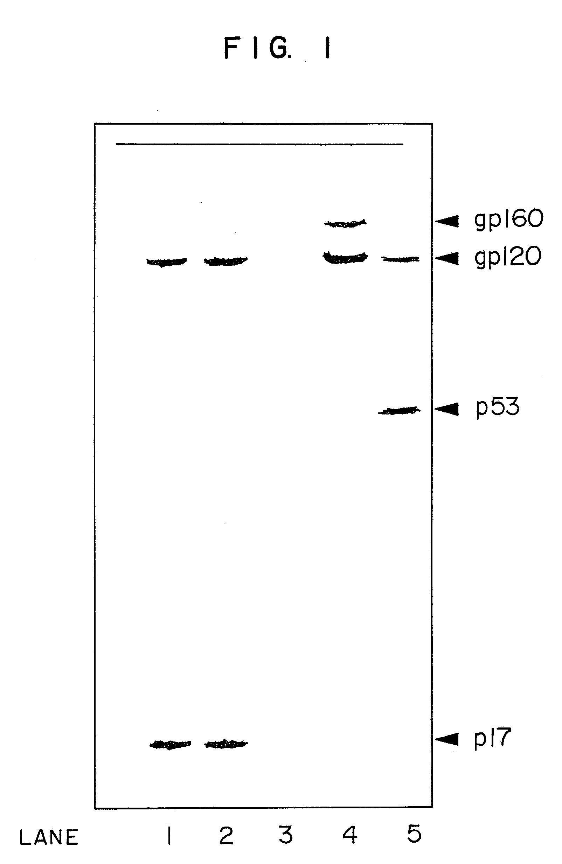

Fig. 1 shows fluorographic images obtained by sodium dodecyl sulfate-containing polyacrylamide gel electrophoresis (SDS-PAGE) of HIV incomplete particles obtained from the culture fluid of persistently HIV-1 infected MT-4 cells according to the present invention.

PREFERRED EMBODIMENTS OF THE INVENTION

The HIV incomplete particles of the present invention comprise no or little protein encoded by gag gene and/or protein encoded by pol gene required for natural poliferation of HIV in host cells. On the other hand, the HIV incomplete particles of the present invention comprise protein encoded by env gene and/or fragments thereof having immunogenicity and are produced by cells in the form of particles of doughnut or fragments thereof. In view of the above properties as material, the HIV incomplete particles of the present invention are obviously different from attenuated or inactivated HIV or compositions such as liposome carring HIV constituents which cannot be produced by cells.

The env gene, gag gene and pol gene as used herein are a part of RNA gene for HIV. The protein encoded by gag gene refers to protein required to construct the "core" structure in the inside of HIV, namely, p 17 or p 24. The protein of p 17 or p 24 is formed from p 53 which is gag precursor protein. The protein encoded by pol gene refers to reverse transcriptase, protease and integrase required to synthesize cDNA corresponding to RNA of HIV in host cells. The protein encoded by env gene refers to envelope protein of HIV having high immunogenicity, which protein includes gp 160 as env precursor protein. Further gp 160 and fragments thereof are glycoproteins present in the envelope protein. The fragments of gp 160 refer to gp 120 and gp 41. It is considered that gp 120 and gp 41 are usually synthesized as components of gp 160 and then separated from each other. Furthermore, the p 53-like substance, gp 160-like substance and gp 120-like substance refer to glycoproteins that undergo electrophoresis to shift near the positions where p 53, gp 160 and gp 120 move and are reactive with anti-HIV antibody in a similar manner.

Next, the HIV incomplete particles of the present invention will be described in more detail.

As a cell line that causes cell death over a wide range after infected with HIV, there are known MT-4 cells [Miyoshi, I. et al., "Type C-virus particles in a cord T-cell line derived by co-cultivating normal human cord leuckocytes and human leukaemic Tcells", Nature, 294, 294, 770-771 (1981)] which are derived from human T-lymphocyte and transformed with HTLV-1. Paying attention to extremely small amounts of live cells found in HIV-1- infected MT-4 cells, the present inventors have continued cultivation to proliferate these cells. As a result, the present inventors have finally succeeded in proliferating these persistently HIV-1-infected cells (MT-4/HIV-1). These cells were further subjected to cloning, whereby many cell clones could be obtained. These cell clones were differentiated into 8 types (I to VIII) in terms of the HIV-1 protein synthesis, reverse transcriptase activity, and HIV-1 infectivity etc. Similar clones were obtained even though either HTLV-IIIB or LAV-I was used. Some of these clones can produce HIV incomplete particles of the present invention in the culture supernatant. In particular, one of the cell clones named by cl-2, which belongs to type IV, produced a large amount of uninfectious doughnut particles which were mainly composed of env gp 120 and gag p 53 protein.

Properties of the thus produced HIV-1 incomplete particles were analyzed. The results are shown in Fig. 1. The analysis was carried out as follows. The culture supernatant of cells labeled with $^{35}$S-cysteine was separated into a fraction containing complete HIV particles alone and a fraction containing complete HIV particles and HIV incomplete particles, by ultracentrifugation using a 25% sucrose phase or by ultracentrifugation using no sucrose phase. Serum from the patient infected with HIV-1 containing both antibodies capable of reacting with p 17 and gp 120 were reacted with the respective fractions. Then, immuno- precipitates were dissolved and the solution was subjected to sodium dodecyl sulfate-containing polyacrylamide gel electrophoresis (SDS-PAGE). Fig. 1 shows its fluorographic images. For control, similar treatments were conducted using MOLT-4 cells [Minowada, J. et al., "Rosette forming lymphoid cell lines. I. Establishment and evidence for origin of thymus-derived lymphocytes", J. Natl. Cancer Inst., 49, 891-895 (1972)] persistently infected with HIV-1 which were releasing infectious complete HIV-1. In Fig. 1, Lanes 1 and 2 indicate results on MOLT-4 cells and Lanes 3 and 4 indicate results on MT-4/HIV-1 cells obtained by the present invention. Lane 5 indicates results on cl-2 cells. Further, Lanes 1 and 3 show the results of particles purified by ultracentrifugation with 25% sucrose, and Lanes 2, 4 and 5 show the results particles purified by ultracentrifugation without 25% sucrose. As is confirmed with Lane 3, p 17, p 53, gp 120 or gp 160-like substance was observed at only trace amount. From this fact, it has been made clear that the clone obtained in the present invention produce predominantly HIV-1 incomplete particles. Further from the results of Lane 4, it has also been made clear that the HIV-1 incomplete particles comprise a gp 120-like substance capable of reacting with an antibody to gp 120 and a gp 160-like substance considered to be a precursor of the gp 120-like substance. As is confirmed with Lane 5, one of HIV-1 incomplete particles according to the present invention comprises gp 120 and p 53.

Furthermore, the HIV-1 incomplete particles were examined by another method as to if they had reverse transcriptase. Reverse transcriptase activity was assayed according to the method of Lee, M. H., et al., "Sensitive reverse transcriptase assay to detect and quantitate human immunodeficiency virus", J. Clin. Microbial., 25, 1717-172 (1987). Very low or no activity was observed in the culture fluid of the cell clones of the present invention. Therefore, it has been made clearer that the HIV-1 incomplete particles do not comprise or do not substantially comprise protein encoded by gag gene and/or protein encoded by pol gene. This is also supported from electron microscopic observation that the doughnut particles were only or predominantly observed for the HIV-1 incomplete particles. However, the HIV incomplete particles of the present invention are not required that both proteins encoded by gag gene and pol gene should be always deleted. The present invention also includes a case in which the HIV incomplete particles do not comprise either protein or scarcely comprise both proteins. The case in which the HIV incomplete particles do not comprise or do not substantially comprise as used herein refers to a protein content to such an extent that cannot exhibit infectivity inherently possessed by HIV. Needless to say, the form of the HIV incomplete particles may be particulate or fragments thereof.

The results explained hereinabove were obtained by MT-4 cell-derived clone established after infection with HTLV-IIIB out of HIV. Similar results were obtained even in the case infected with LAV. That is, similar HIV incomplete particles can be obtained, irrespective of kind of HIV used. On the other hand, MOLT-4 cells generally produce chiefly complete HIV particles but a possibility of producing HIV incomplete particles cannot be denied. Accordingly, it cannot be denied that clone capable of releasing HIV incomplete particles might be isolated from MOLT-4 cells by an appropriate treatment.

The infectivity of the HIV-1 incomplete particles in accordance with the present invention is not greater than $10^{0.8}TCID_{50}$/ml, which failed to detect even by the fluorescent antibody method. The infectivity was determined according to the method of Ikuta, K. et al., "Amplification of human immunodeficiency virus production from a virus-producing cell line in culture at high cell density", Jpn. J. Cancer Res. (Gann), 78, 643-647 (1987). Therefore, it was judged that the infectivity of the HIV-1 incomplete particles was completely negative or substantially negative.

To recover the HIV incomplete particles from the culture fluid of HIV incomplete particle-producing cells, various methods are applicable. This purpose can be achieved by, for example, a method of fractionating the HIV incomplete particles from the culture fluid of the cells by ultracentrifugation, a method of molecular sieve or ultrafiltration, etc. or in appropriate combination of these methods.

The HIV incomplete particles of the present invention can be used as HIV vaccine free from infectivity and having high immunogenicity. In addition, the HIV incomplete particles are also utilizable as antigen for assaying HIV antibody in serum, for screening of anti-viral agents and as a carrier for injecting a drug into HIV-infected cells, paying attention to binding activity to cells.

Example 1

MT-4 cells were cultured in 10% bovine fetal serum-containing RPMI-1640 medium. When the cells grew to $1 \times 10^6$ cells/ml, HIV-1 (HTLV-IIIB) was infected in 0.1 m.o.i. Four days after, more than 90% of the cells were denatured and came to death but 15 days after, growth of survived cells was noted. About one month after, the cells showed proliferation close to the original MT-4 cells. After serial cultivation for additional 2 months, cloning was performed by limiting dilution in 96 well-microplates or colony-formation in soft-agar in 60 mm diameter dishes. Eight types of cell clones were obtained. The HIV-1 incomplete particles released from these clones into the culture fluid did not pass through a 25% sucrose solution phase by centrifugation at 48 Krpm for 2 hours and precipitates were recovered by centrifugation at 48 Krpm for an hour. The cell clones of Types VII and VIII synthesized env protein (gp 120 and gp 160), but not gag protein and pol protein. Such clones produced the HIV-1 incomplete doughnut particles comprise env protein as main component, but not gag protein and pol protein. The cell clones of Types IV and V synthesized env protein and gag protein, but not pol protein. In particular, Type IV cell clones (cl-2) synthesized env protein and gag protein, but did not nature gag protein. Therefore, the cl-2 cells produce env protein (gp 120) and uncleaved gag p 53, but not pol protein. The infectivity of the HIV-1 incomplete particles recovered was less than the detectable limit by the fluorescent antibody method. In the immunoprecipitation method, no protein capable of reacting with anti-gp 17 antibody was noted but protein capable of reacting with anti-gp 120 antibody. According to an electron microscopic observation, doughnut particles in which no "core" structure was noted could be confirmed.

The HIV incomplete particles according to the present invention have no infectivity and are extremely similar in their constituents and structure to the envelope of naturally occurring HIV. Moreover, the HIV incomplete particles are expected to exhibit higher immunogenicity than the conventional liquid vaccines prepared by recombinat DNA technology. Therefore, the HIV incomplete particles are safe and expected to exhibit high immunogenicity similar to the natural HIV envelope. Accordingly, the HIV incomplete particles of the present invention are expected to be effective as drugs such as vaccine, etc. for preventing onset of, e.g., AIDS.

**Claims**

1. HIV incomplete particles which comprise protein encoded by env gene and/or fragments thereof as main constituent protein, do not comprise or do not substantially comprise protein encoded by gag gene and/or protein encoded by pol gene and are produced by cells.

2. HIV incomplete particles according to Claim 1, which comprises as main constituent protein gp 120 or a gp 120-like substance, gp 160 or a gp 160-like substance, and/or fragments thereof.

3. HIV incomplete particles according to Claim 1, wherein said cells are those survived after infected with HIV.

4. HIV incomplete particles according to Claim 1, wherein said cells are MT-4/HIV-1 cells which correspond to MT-4 cells survived after infected with HIV-1.

5. HIV incomplete particles according to Claim 4, wherein HIV-1 is HTLV-IIIB or LAV-I.

6. HIV incomplete particles according to Claim 1, which comprise the gp-160 or gp 160-like substance, and gp 120 or the gp 120-like substance.

7. HIV incomplete particles according to Claim 1, which do not comprise or do not substantially comprise both protein encoded by gag gene and protein encoded by pol gene.

8. HIV incomplete particles according to Claim 1, which comprises p 53 or a p 53-like substance, but not protein encoded by pol gene.

9. A method for preparing HIV incomplete particles which comprises infecting cells with HIV, harvesting cells survived thereafter and capable of producing HIV incomplete particles, culturing the cells and recovering the HIV incomplete particles from the culture fluid.

10. A method according to Claim 9, wherein said cells are MT-4 cells.

11. A method according to Claim 9, wherein HIV is HIV-1.

12. A method according to Claim 11, wherein HIV-1 is HTLV-IIIB or LAV-I.

# F I G. 1